# EUROPEAN PATENT APPLICATION

(11) **EP 4 685 467 A1**
(43) Date of publication of application: **28.01.2026**
(21) Application number: 25190508.9
(22) Date of filing: 18.07.2025
(51) Int. Cl.: G01N 21/78

(54) **ANALYSIS APPARATUS**

(30) Priority: 23.07.2024 JP 2024117840
(71) Applicant: FUJIFILM Corporation, Tokyo 106-8620 (JP)
(72) Inventor: MIURA, Yoshinobu, Tokyo, 106-8620 (JP)
(74) Representative: Dehns Germany Partnerschaft mbB

(57) **Abstract**

An analysis apparatus (100) includes: a support section (62) that supports, at a measurement position, an analysis chip (12) having a reaction region (12A), which holds a reagent, and used for quantitative analysis of reaction with a detection target substance that reacts with the reagent; a photometry unit (70) including a light-emitting element that irradiates the reaction region of the analysis chip with measurement light for measuring the reaction, and an area sensor that captures an image of a predetermined imaging range including the reaction region irradiated with the measurement light; and a color plate (75) that is arranged within the imaging range, that has a region irradiated with the measurement light, that has characteristics in which a reflectivity changes in accordance with a wavelength of incident light of the measurement light, and that is used for detecting a wavelength variation of the measurement light.

## Description

### BACKGROUND OF THE INVENTION

### 1. Field of the Invention

The present disclosure relates to an analysis apparatus.

### 2. Description of the Related Art

An analysis apparatus that analyzes a specimen sample using an analysis chip on which the specimen sample is spotted is known. In the analysis of the specimen sample, a concentration of a detection target substance contained in the specimen sample is measured by measuring a reaction state between the specimen sample and a reagent. The specimen sample is, for example, blood or urine. As the analysis chip, an analysis chip comprising a reaction region containing a dry reagent is generally used.

In the analysis apparatus, a reaction product generated by the reaction between the detection target substance and the reagent is detected by irradiating a reaction region, on which the specimen sample is added dropwise, of such an analysis chip with measurement light and detecting reflected light thereof. Therefore, the analysis apparatus comprises a photometry unit that irradiates the analysis chip with the measurement light and detects the reflected light. Then, in the analysis apparatus, an optical density of a reflection region is obtained from an amount of the reflected light, and the detection target substance is quantitatively analyzed from the optical density.

JP2004-132706A proposes a method of suppressing a decrease in measurement accuracy due to a variation in a central wavelength of a light source in an analysis apparatus.

### SUMMARY OF THE INVENTION

In JP2004-132706A, a reaction state between the specimen sample and the reagent is measured, a measurement wavelength is sequentially detected, and quantitative analysis is performed using a calibration curve in accordance with the measurement wavelength. In JP2004-132706A, since the measurement of the reaction state and the detection of the measurement wavelength are sequentially performed, in a case in which the central wavelength of the light source changes between the two measurements, the analysis accuracy is lowered.

The technology of the present disclosure has been made in view of the above-described circumstances, and an object of the present disclosure is to provide an analysis apparatus that can perform quantitative analysis with higher accuracy than in the related art.

According to an aspect of the present invention, there is provided an analysis apparatus comprising: a support section that supports, at a measurement position, an analysis chip having a reaction region, which holds a reagent, and used for quantitative analysis of a detection target substance that reacts with the reagent; a photometry unit including a light-emitting element that irradiates the reaction region of the analysis chip with measurement light for measuring the reaction, and an area sensor that captures an image of a predetermined imaging range including the reaction region irradiated with the measurement light; and a color plate that is arranged within the imaging range, that has a region irradiated with the measurement light, that has characteristics in which a reflectivity changes in accordance with a wavelength of incident light, and that is used for detecting a wavelength variation of the measurement light.

It is preferable that a central wavelength of the measurement light is in a wavelength range of 400 nm to 700 nm, and the color plate is used for detecting the wavelength variation of the measurement light emitted from the light-emitting element.

It is preferable that the color plate has an optical density that changes by 0.6 or more in the wavelength range.

It is preferable that the color plate has the optical density that changes by 0.2 or more in a region of ±40 nm with respect to a central wavelength of the light-emitting element.

A plurality of light-emitting elements that emit the measurement light in wavelength ranges different from each other may be provided as the light-emitting elements, and the color plate may include a plurality of color plates of which the optical density changes in accordance with the wavelength range of each of the plurality of light-emitting elements.

The color plate may include a color plate of which the optical density changes by 0.2 or more in a wavelength range of 400 nm to 450 nm.

The color plate may include a color plate of which the optical density changes by 0.2 or more in a wavelength range of 500 nm to 580 nm.

The color plate may include a color plate of which the optical density changes by 0.2 or more in a wavelength range of 600 nm to 680 nm.

In a case in which the color plate is defined as a first color plate, a second color plate different from the first color plate may be provided, and it is preferable that the second color plate is arranged within the imaging range, has a region irradiated with the measurement light emitted from the light-emitting element, and has an optical density that changes by less than 0.2 in a wavelength range of 400 nm to 700 nm.

It is preferable that the optical density of the second color plate is 1.5 or less.

It is preferable that the analysis chip contains a dry reagent as the reagent.

It is preferable that the analysis apparatus further comprises: a processor that acquires the image from the photometry unit to perform the quantitative analysis of the detection target substance based on a measurement value corresponding to a photometric region brightness value that is a brightness value of the reaction region extracted from the acquired image, in which the processor detects the wavelength variation of the measurement light based on the image to perform the quantitative analysis in accordance with the detected wavelength variation.

With the analysis apparatus according to the present disclosure, even in a case in which an illuminance distribution varies, the quantitative analysis can be performed with high accuracy.

### BRIEF DESCRIPTION OF THE DRAWINGS

Fig. 1 is a schematic diagram showing an overall configuration of an analysis apparatus according to an embodiment.
Fig. 2 is a plan view showing a main part of the analysis apparatus of Fig. 1.
Fig. 3 is a diagram showing a configuration example of an analysis chip.
Fig. 4 is a schematic diagram showing a positional relationship between a schematic configuration of a photometry unit and the analysis chip.
Fig. 5 is a perspective view showing a positional relationship between a main part of the photometry unit and the analysis chip and a color plate.
Fig. 6 is a schematic diagram showing a spectral reflectivity curve of the color plate.
Fig. 7 is a diagram showing a spectral reflectivity curve of a specific color plate.
Fig. 8 is a diagram showing an image captured by an area sensor.
Fig. 9 is a perspective view showing a positional relationship between the main part of the photometry unit, the analysis chip, a first color plate, and a second color plate.
Fig. 10 is a diagram showing an image captured by an area sensor in a case in which the second color plate is provided.
Fig. 11 is a diagram showing images P11 to P16 captured by applying current values of 0 mA, 6 mA, 9 mA, 15 mA, 19 mA, and 39 mA to a light-emitting element.
Fig. 12 is a diagram showing a relationship between a light-emitting element illuminance and a pre-correction brightness value.
Fig. 13 is a diagram showing a relationship between the light-emitting element illuminance and a pre-photometric region brightness value.
Fig. 14 is a diagram showing a correlation between the pre-correction brightness value and the pre-photometric region brightness value.

### DESCRIPTION OF THE PREFERRED EMBODIMENTS

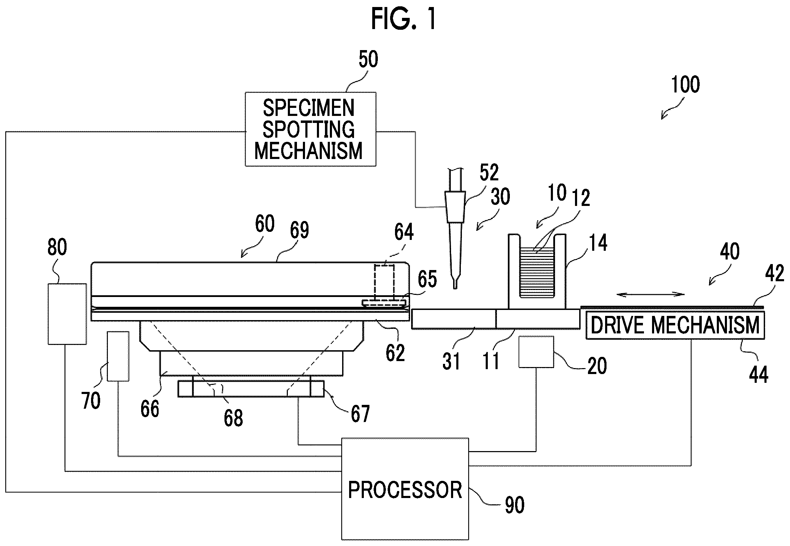

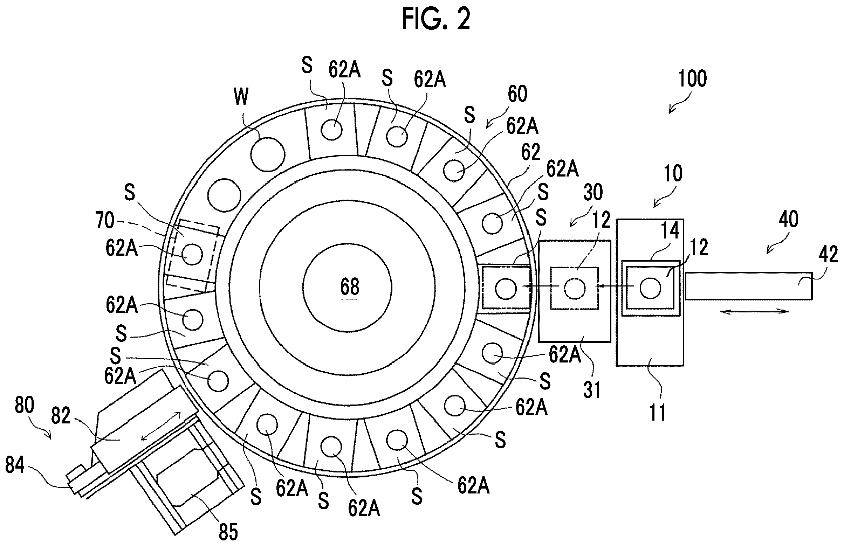

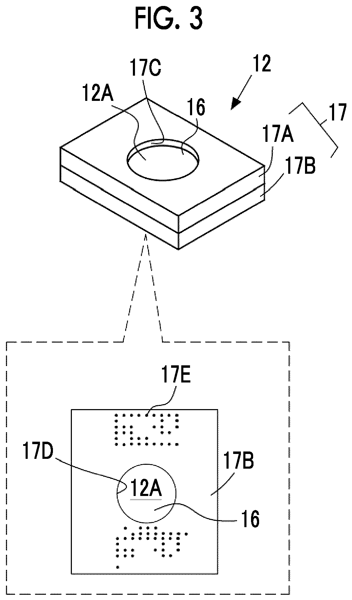

Hereinafter, preferred embodiments of the present disclosure will be described with reference to the drawings. In the drawings, the same reference numerals are given to the same components. Fig. 1 is a schematic diagram showing an overall configuration of an analysis apparatus 100 according to the embodiment. Fig. 2 is a plan view showing a main part of the analysis apparatus 100 of Fig. 1, and Fig. 3 is a diagram showing a configuration example of an analysis chip.

The analysis apparatus 100 shown in Fig. 1 is an example of an analysis apparatus that analyzes a specimen sample. An analysis chip 12 is attachably and detachably loaded into the analysis apparatus 100. In the analysis apparatus 100, for example, a dry analysis chip is used, and a concentration of a detection target substance contained in the specimen sample is measured. Specifically, the analysis apparatus 100 quantitatively determines the concentration of the detection target substance by colorimetric measurement. The specimen sample is, for example, plasma, whole blood, serum, or urine.

As shown in Fig. 3, the analysis chip 12 has a planar reaction region 12A in which a reagent is fixed. The reagent reacts with the detection target substance to generate a substance that develops a specific color. The substance that develops color by this reaction is hereinafter referred to as a reactant. As the reagent, for example, a dry reagent that is in a dry state at least during shipment is used. The specimen sample is spotted onto the reaction region 12A of the analysis chip 12.

More specifically, the analysis chip 12 has a carrier 16 including the reaction region 12A on which the specimen sample is spotted, and the carrier 16 is accommodated in a case 17. The case 17 is composed of a first case 17A and a second case 17B, and accommodates the carrier 16 such that the carrier 16 is sandwiched between the first case 17A and the second case 17B. An opening 17C that functions as a dropwise-addition port for spotting the specimen sample onto the reaction region 12A is formed in the first case 17A. An opening 17D for irradiating the reaction region 12A with light is formed in the second case 17B. The carrier 16 is exposed to the opening 17C of the first case 17A constituting a front surface of the analysis chip 12. In addition, the carrier 16 is exposed to the opening 17D of the second case 17B constituting a back surface of the analysis chip 12. A region exposed to the opening 17D of the carrier 16 constitutes the reaction region 12A in which the reagent is fixed. In addition, item information related to a measurement item is assigned to the second case 17B as information code 17E that is encoded. The information code 17E is, for example, a pattern in which a plurality of dots are arranged, and the arrangement pattern of the dots is varied for each measurement item. It goes without saying that a one-dimensional barcode, a two-dimensional barcode, or the like may be used as the information code 17E.

The analysis apparatus 100 comprises a chip set section 10, a reader 20, a specimen spotting section 30, a chip transport mechanism 40, a specimen spotting mechanism 50, an incubator 60, a photometry unit 70, a chip disposal mechanism 80, and a processor 90.

A stocker 14 that accommodates the analysis chip 12 on a holding table 11 is arranged in the chip set section 10. A plurality of analysis chips 12 are stacked and accommodated in the stocker 14. The stocker 14 has an opening on a bottom surface. The analysis chip 12 is accommodated with a posture in which a surface on which the information code 17E is recorded faces the opening side of the stocker 14. Therefore, in the stocker 14, the information code 17E of the analysis chip 12 located on the lowermost stage on the most opening side is exposed from the opening. In addition, an opening is also formed in the holding table 11 on which the stocker 14 is arranged. Therefore, the information code 17E of the analysis chip 12 located on the lowermost stage in the stocker 14 is exposed to the reader 20 through the opening of the holding table 11 and the opening of the stocker 14. The reader 20 is arranged below the holding table 11 and reads the exposed information code 17E.

The reader 20 is, as an example, a code reader that reads the item information given to the analysis chip 12. The reader 20 is configured with, for example, an image sensor such as a charge-coupled device (CCD) and a complementary metal-oxide-semiconductor (CMOS). The item information read by the reader 20 is output to the processor 90.

The chip transport mechanism 40 transports the analysis chip 12 from the chip set section 10 to the specimen spotting section 30, and further transports the analysis chip 12 from the specimen spotting section 30 to the incubator 60. The chip transport mechanism 40 comprises a thin plate-shaped chip transport member 42 and a drive mechanism 44 that reciprocates the chip transport member 42 in a direction in which the chip set section 10, the specimen spotting section 30, and the incubator 60 are arranged. The drive mechanism 44 is, for example, a linear actuator. The chip transport member 42 is slidably supported by a guide rod (not shown) and is reciprocated by the drive mechanism 44. The chip transport member 42 is pressed against the analysis chip 12 accommodated in the lowermost stage among the analysis chips 12 stacked in the stocker 14. The analysis chip 12 is transported to the incubator 60 side by moving the chip transport member 42 to the incubator 60 side in this state.

In the specimen spotting section 30, the specimen sample such as blood plasma, whole blood, serum, or urine is spotted onto the analysis chip 12. The specimen spotting section 30 is provided with a chip support table 31, and the specimen sample spotting onto the analysis chip 12 transported onto the chip support table 31 is performed on the chip support table 31. The specimen sample is spotted by the specimen spotting mechanism 50 described later. The chip support table 31 is arranged adjacent to the holding table 11.

As shown in Fig. 1, the specimen spotting mechanism 50 comprises a nozzle 52, a suction-and-discharge mechanism (not shown), and a moving mechanism that moves the nozzle 52. The specimen spotting mechanism 50 suctions the specimen sample from a specimen accommodation section (not shown) and spots the specimen sample onto the analysis chip 12 in the specimen spotting section 30.

The incubator 60 can accommodate a plurality of analysis chips 12 inside. The incubator 60 has a constant temperature function of keeping a temperature constant in order to promote the reaction between the reagent and the specimen sample of the analysis chip 12. A set temperature is, for example, 37°C.

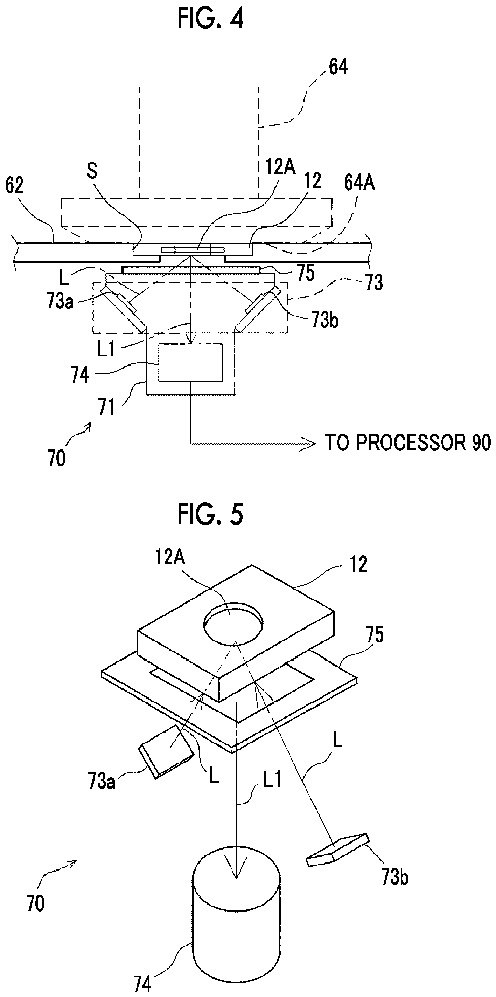

As shown in Fig. 2, the incubator 60 comprises an annular rotary substrate 62 provided with a plurality of cells S in which the analysis chip 12 is loaded. In addition, a disk-shaped holding member 65 is provided above the rotary substrate 62, the holding member 65 including a pressing member 64 that presses the analysis chip 12 loaded in the cell S in a direction facing the reaction region 12A (see Fig. 3). The pressing member 64 is provided corresponding to each cell S. A slit-shaped space is formed between a pressing surface 64A (see Fig. 4) of the pressing member 64 and the cell S, and the analysis chip 12 is loaded herein. The cells S are sequentially transported to a measurement position at which the photometry unit 70, which will be described later, is arranged, by rotating the rotary substrate 62. The rotary substrate 62 is an example of a support section that supports the analysis chip 12 at the measurement position.

A rotary cylinder 66 is provided below the rotary substrate 62. The rotary cylinder 66 has a substantially inverted triangular cross-sectional shape of which an inner diameter decreases downward. A bearing 67 is arranged below an outer periphery of the rotary cylinder 66, and the rotary cylinder 66 is rotatably supported by the bearing 67. The rotary substrate 62 rotates as the rotary cylinder 66 rotates. It should be noted that the holding member 65 rotates integrally with the rotary substrate 62. The rotary cylinder 66 has an opening at a bottom portion as a vertex portion of an inverted triangle, and this opening functions as a disposal hole 68 for disposing of the used analysis chip 12. The used analysis chip 12 is moved to the center side of the annular rotary substrate 62 from a state of being loaded in the cell S, and is dropped toward an inclined surface of the rotary cylinder 66. The used analysis chip 12 dropped into the rotary cylinder 66 slides on the inclined surface and is disposed of from the disposal hole 68.

A heating unit such as a heater (not shown) is arranged in the holding member 65, and the analysis chip 12 accommodated in the cell S is kept constant at a predetermined temperature by the temperature adjustment. A thermal insulation cover 69 is arranged on an upper surface of the holding member 65. It should be noted that Fig. 2 shows a state in which the holding member 65 and the thermal insulation cover 69 are removed and the rotary substrate 62 is exposed.

As shown in Fig. 2, an opening window 62A for photometry is formed at the center of the bottom surface of each cell S of the rotary substrate 62, and the colorimetric measurement of the analysis chip 12 is performed by the photometry unit 70 arranged below the rotary substrate 62 through the opening window 62A.

The photometry unit 70 performs the colorimetric measurement, which is the measurement of optical density using a colorimetric method, on the analysis chip 12. The photometry unit 70 is provided below the rotary substrate 62 in an outer peripheral portion of the incubator 60. The photometry unit 70 acquires a detection signal representing the optical density of the reaction region 12A of the analysis chip 12, and outputs the detection signal to the processor 90.

Fig. 4 is a diagram showing a positional relationship between a schematic configuration of the photometry unit 70 and the analysis chip 12 during the measurement. As shown in Fig. 4, the photometry unit 70 comprises a housing 71, an irradiation device 73 including light-emitting elements 73a and 73b that irradiate the reaction region 12A with measurement light L, and an area sensor 74 that images the reaction region 12A.

It should be noted that the housing 71 comprises an optical system (not shown) for condensing reflected light L1 from the reaction region 12A and guiding the reflected light L1 to the area sensor 74. In the present example, the light-emitting elements 73a and 73b have substantially the same central wavelength. Here, the central wavelength refers to a central wavelength of light emission spectrum characteristics of the light-emitting element. In a case in which the two light-emitting element 73a and 73b having substantially the same central wavelength are provided as in the present example, the light emission spectra of both the light-emitting elements are added, and the central wavelength of the added light emission spectrum characteristics is defined as the central wavelength in the present disclosure.

The wavelength of the measurement light L is determined in accordance with the detection target substance (that is, the measurement item). For example, in the present example, as described above, the reactant that develops the specific color is generated by the reaction between the detection target substance and the reagent. Since the light emitted from the irradiation device 73 is the measurement light L for detecting whether or not the reactant is generated, the wavelength is determined in accordance with the color developed by the reactant. The measurement light L of the present example is, for example, light including a wavelength absorbed by the reactant in order to detect the reactant. In the present example, a configuration has been described in which the two light-emitting elements having the same central wavelength are provided, but the irradiation device 73 may comprise a plurality of light-emitting elements having different central wavelengths in order to emit the measurement light L having different wavelengths depending on the detection target substance. As the light-emitting elements 73a and 73b, for example, a light-emitting diode (LED), organic electro luminescence (EL), or a semiconductor laser is used.

In a case in which the analysis chip 12 is irradiated with the measurement light L, the area sensor 74 captures an image of a predetermined imaging range including the reaction region 12A of the analysis chip 12. The area sensor 74 is, for example, an image sensor such as a CCD camera or a CMOS camera. The area sensor 74 outputs the captured image to the processor 90.

Further, as shown in Fig. 4, a color plate 75 is arranged within the imaging range of the area sensor 74. Fig. 5 is a perspective view showing a positional relationship between a main part of the photometry unit 70 and the color plate 75 and the analysis chip 12 during the measurement. In Fig. 5, the rotary substrate 62 is not shown. The color plate 75 is arranged between the rotary substrate 62 and the photometry unit 70. The color plate 75 has a region irradiated with the light (measurement light L) emitted from the light-emitting elements 73a and 73b. In the present example, the color plate 75 is a rectangular member having a rectangular opening in a center portion. The measurement light L emitted from the light-emitting elements 73a and 73b passes through the opening of the color plate 75 and is incident on the reaction region 12A. It should be noted that the color plate 75 being arranged within the imaging range of the area sensor 74 does not mean that the entire color plate 75 is arranged within the imaging range, and at least a part of the color plate 75 need only be arranged within the imaging range. The color plate 75 has characteristics in which the optical density changes in accordance with the wavelength of the measurement light L, and is used for detecting a wavelength variation of the measurement light L.

Here, the wavelength of the measurement light L refers to a central wavelength of the measurement light L, and means a central wavelength of the light emitted from the light-emitting elements 73a and 73b. The color plate 75 need only be able to detect a deviation of the central wavelength of the measurement light L, and may be any color plate as long as the reflectivity R changes before and after the wavelength of the measurement light L. It should be noted that, since an optical density OD and a reflectivity R have a relationship of OD = log₁₀(1/R), "the reflectivity changes" means "the optical density changes".

In general, the central wavelength of the measurement light L is preferably in a wavelength range of a visible range of a wavelength of 400 nm to 700 nm, and the color plate 75 is used for detecting the wavelength variation of the measurement light L emitted from the light-emitting elements 73a and 73b. Here, the "detection of the wavelength variation" may be detection of a deviation of an initial central wavelength of the measurement light L, or may be detection of the central wavelength of the measurement light L itself during the measurement. The "initial central wavelength" is, for example, a central wavelength presented by a manufacturer of the light-emitting elements 73a and 73b at the time of shipment.

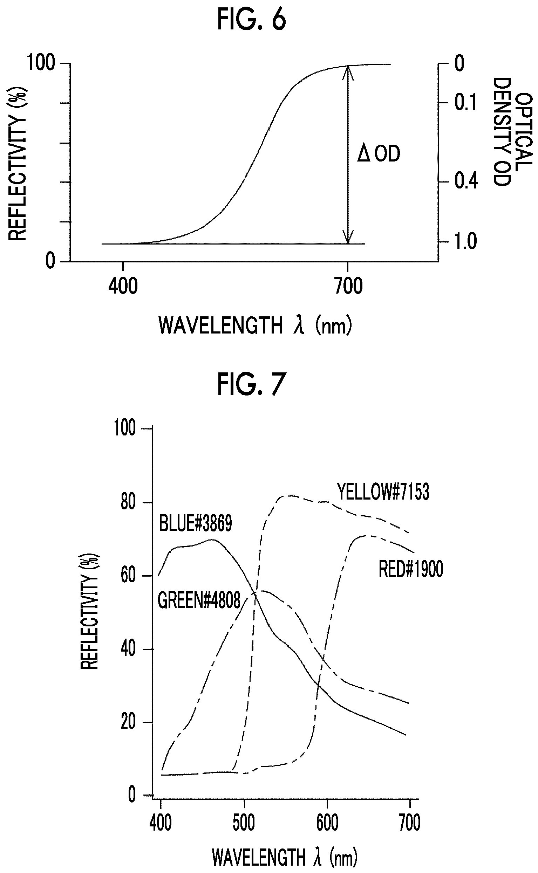

As shown in Fig. 6, the color plate 75 is, for example, a color plate of which the optical density OD changes by 0.6 or more in a wavelength range of 400 nm to 700 nm. Here, the fact that the optical density OD changes by 0.6 or more means that, as shown in Fig. 6, a difference ΔOD between a maximum value and a minimum value of the optical density in the wavelength range of 400 nm to 700 nm is 0.6 or more.

In the color plate 75, it is preferable that the optical density changes by 0.2 or more in a region of ±40 nm with respect to the initial central wavelength of the light-emitting elements 73a and 73b. In the color plate 75, it is more preferable that the optical density changes by 0.2 or more in a region of ±20 nm with respect to the central wavelength of the light-emitting elements 73a and 73b.

For example, in a case in which the measurement light L is blue light having a wavelength of about 420 nm to 430 nm, it is preferable that, as the color plate 75, a color plate of which the optical density changes by 0.2 or more in a wavelength range of 400 nm to 450 nm is provided. In a case in which the measurement light L is green light having a wavelength of about 530 nm to 550 nm, it is preferable that, as the color plate 75, a color plate of which the optical density changes by 0.2 or more in a wavelength range of 500 nm to 580 nm is provided. Further, in a case in which the measurement light L is red light having a wavelength of about 630 nm to 650 nm, it is preferable that, as the color plate 75, a color plate of which the optical density changes by 0.2 or more in a wavelength range of 600 nm to 680 nm is provided.

In a case in which the irradiation device 73 comprises a plurality of light-emitting elements having different central wavelengths in order to emit the measurement light L having different wavelengths depending on the detection target substance, it is preferable that a plurality of color plates of which the optical density changes in accordance with the wavelength range of each of the plurality of light-emitting elements are provided as the color plates 75. For example, in a case in which a light-emitting element that emits blue light having a wavelength of about 420 nm to 430 nm and a light-emitting element that emits red light having a wavelength of about 630 nm to 650 nm are provided, a color plate of which the optical density changes by 0.2 or more in a wavelength range of 400 nm to 450 nm and a color plate of which the optical density changes by 0.2 or more in a wavelength range of 600 nm to 680 nm are provided as the color plates 75.

Fig. 7 shows spectral reflectivity curves for the four color plates (extracted from the spectral reflectivity curve described in homepage of EVERS CORPORATION [https://evers.c.ooco.jp/] (search date: July 22, 2024)).

A color plate of BLUE #3869 is suitable for the measurement light L having a central wavelength of 500 nm to 650 nm. A color plate of GREEN #4808 is suitable for the measurement light L having a central wavelength of 400 nm to 480 nm or 550 nm to 650 nm. A color plate of YELLOW#7153 is suitable for the measurement light L having a central wavelength of 500 nm to 530 nm. A color plate of RED#1900 is suitable for the measurement light L having a central wavelength of 600 nm to 620 nm. The color plate 75 need only be provided as appropriate in accordance with the central wavelengths of the light-emitting elements 73a and 73b provided in the irradiation device 73.

The detection of the wavelength variation using the color plate 75 will be described below.

The chip disposal mechanism 80 comprises a thin plate-shaped chip transport member 82 and a drive mechanism 84 that reciprocates the chip transport member 82. The chip disposal mechanism 80 inserts the chip transport member 82 into the cell S from the outer peripheral portion of the incubator 60 and pushes the used analysis chip 12 after the measurement to the central portion of the incubator 60 to drop the used analysis chip 12 into the disposal hole 68. The drive mechanism 84 is, for example, a linear actuator. The chip transport member 82 is slidably supported by a guide rod (not shown) and is reciprocated by the drive mechanism 84. It should be noted that a collection box for collecting the used analysis chip 12 is arranged below the disposal hole 68.

The analysis chip 12 is loaded into a slit-shaped space formed between the cell S and the pressing member 64 of the rotary substrate 62 in the incubator 60. The analysis chip 12 is warmed in the incubator 60, and is transported to the measurement position by the rotation of the incubator 60. The measurement position is a position at which the photometry unit 70 is arranged below the rotary substrate 62 and the colorimetric measurement of the analysis chip 12 is performed. The analysis chip 12 on which the colorimetric measurement is performed by the photometry unit 70 is dropped into the disposal hole 68 and is disposed of by the chip disposal mechanism 80.

The processor 90 integrally controls the respective units of the analysis apparatus 100. The configuration of the processor 90 is not particularly limited, but for example, the processor 90 is configured with a central processing unit (CPU), a non-volatile memory (NVM), a random-access memory (RAM), and the like.

The processor 90 acquires the image captured by the area sensor 74 of the photometry unit 70 from the area sensor 74 and performs the quantitative analysis of the detection target substance contained in the specimen sample based on the acquired image.

The processor 90 performs the quantitative analysis of the detection target substance based on a measurement value corresponding to a photometric region brightness value that is a brightness value of the reaction region 12A extracted from the image acquired from the photometry unit 70. Specifically, the processor 90 derives the optical density of the reaction region 12A as the measurement value, and derives the concentration of the detection target substance based on a calibration curve showing a relationship between the optical density and the concentration of the detection target substance. In this case, the processor 90 detects the wavelength variation of the measurement light based on the image, corrects the measurement value depending on the detected wavelength variation, and derives the concentration of the detection target substance using the corrected measurement value. Here, "deriving the concentration of the detection target substance" means quantifying the detection target substance.

The processor 90 corrects the wavelength variation based on a relationship between the wavelength variation and a light amount variation stored in a memory in advance. Specifically, the correction is performed in the following manner.

In the analysis apparatus 100, a relationship between a wavelength and a reflectivity of the incident light of the color plate 75 acquired in advance is provided in the memory. For example, the relationship shown in Fig. 6 described above is provided in the memory. The reflectivity is derived from the brightness value in the image captured by the area sensor 74. The "relationship between the wavelength and the reflectivity of the incident light" may be a relationship between a parameter related to the reflectivity such as the brightness value or the optical density of the image, instead of the "reflectivity" itself, and the wavelength of the incident light. Then, for example, a plurality of calibration curves are provided in the memory, which represent a relationship between the optical density of the reaction region 12A and the specimen density in a case in which the measurement is performed at each wavelength that is ±3 nm of the wavelength of the measurement light L. That is, in a case in which the initial central wavelength of the measurement light L is 500 nm, the memory comprises, for example, a calibration curve acquired with light having a central wavelength of 500 nm, and calibration curves acquired with light having central wavelengths of 497 nm, 498 nm, 499 nm, 501 nm, 502 nm, and 503 nm.

In a case of the colorimetric measurement of the analysis chip 12, that is, in a case in which the analysis chip 12, in which the specimen sample is added dropwise to the reaction region 12A, is used for the measurement to perform the quantitative analysis of the detection target substance in the specimen sample, the processor 90 performs the following processing.

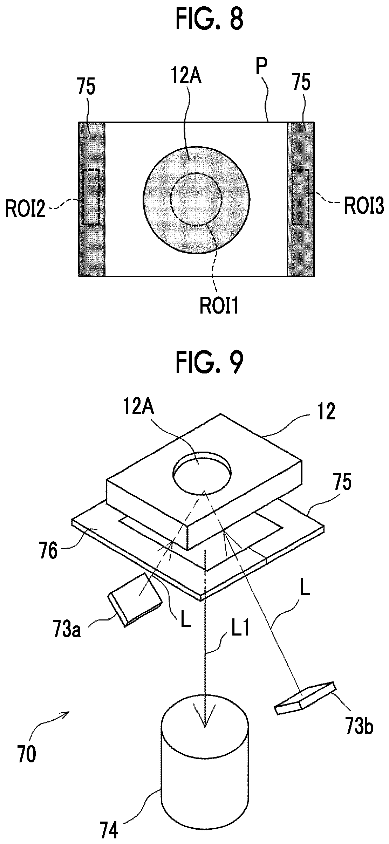

As shown in Fig. 4, the light-emitting elements 73a and 73b are turned on to irradiate the reaction region 12A and the color plate 75 with the measurement light L, and in this state, the area sensor 74 captures the image including the reaction region and the color plate 75. The processor 90 acquires the image from the area sensor 74. Fig. 8 is a schematic diagram showing an image P acquired from the photometry unit 70 by the processor 90. In the image P, an outer shape of a circular portion at the center is an outer shape of the opening window 62A of the rotary substrate 62. An inner portion of the circular portion is the reaction region 12A of the analysis chip 12 observed from the opening window 62A. In addition, members indicated by gray and located on both sides of the image P are the color plates 75. In this way, the image P includes the reaction region 12A and at least a partial region of the color plate 75.

The processor 90 sets a predetermined region in the reaction region 12A, for example, a central portion of the reaction region 12A shown in Fig. 8 as a region of interest ROI1, and derives an average value A of brightness data in this range as a photometric region brightness value (hereinafter, a measurement value A). Then, the processor 90 sets two regions of interest ROI2 and ROI3 of the color plate 75 as a region for extracting wavelength variation detection brightness data, and derives an average value C of the brightness data in this range as a wavelength variation detection brightness value. The processor 90 derives the reflectivity from the wavelength variation detection brightness value, and specifies the wavelength of the measurement light L from the relationship between the wavelength and the reflectivity of the incident light (see Fig. 6). As a result, the wavelength of the measurement light L during the analysis is specified with high accuracy. The processor 90 selects the calibration curve measured at the specified wavelength and quantifies the detection target substance from the measurement value A by using the selected calibration curve.

As described above, the analysis apparatus 100 according to the present embodiment comprises the photometry unit 70 including the light-emitting elements 73a and 73b that irradiate the reaction region 12A of the analysis chip 12 with the measurement light L for measuring the reaction, and the area sensor 74 that captures the image of a predetermined imaging range including the reaction region 12A irradiated with the measurement light L, and the color plate 75 that is arranged within the imaging range, that has a region irradiated with the measurement light L, that has characteristics in which the reflectivity changes in accordance with the wavelength of the incident light, and that is used for detecting the wavelength variation of the measurement light L. With the above-described configuration, during the analysis, the area sensor 74 can capture the image including the reaction region 12A and the color plate 75, and the measurement of the brightness value of the reaction region 12A and the measurement of the brightness value of the color plate 75 can be performed simultaneously without a time difference. In a case in which the measurement of the coloring reaction of the reaction region 12A and the detection of the wavelength variation of the measurement light L are sequentially performed, the central wavelength of the light-emitting elements 73a and 73b may change between the two measurements. In a case in which the variation in the central wavelength occurs between the measurement of the reaction region 12A and the detection of the wavelength variation, the analysis accuracy is lowered. On the other hand, in the present embodiment, since the two measurements can be performed at the same time, a wavelength deviation does not occur between the measurement of the reaction region and the wavelength detection of the measurement light, and the analysis accuracy can be prevented from being lowered as compared with a case in which the measurements are sequentially performed, and the quantitative analysis can be performed with higher accuracy than in the related art.

In the colorimetric measurement, in a case in which the wavelength of the measurement light L changes, the amount of the reflected light from the reaction region 12A, that is, the brightness in the image may change. Therefore, in a case in which the wavelength variation is not taken into consideration, the accuracy of the quantified density of the detection target substance is lowered. As in the above-described embodiment, the actual central wavelength of the measurement light is specified, and the calibration curve corresponding to the wavelength is used, whereby accurate quantitative analysis can be performed.

In the above-described embodiment, the reflectivity is derived from the brightness data of the color plate 75 in the image P at the time of analysis, the central wavelength of the measurement light L is specified, and the quantification of the detection target substance is performed using the calibration curve acquired by the light having the central wavelength. However, the method of detecting the wavelength variation of the measurement light and performing the quantitative analysis in accordance with the detected wavelength variation is not limited to the above-described method. The central wavelength of the measurement light L may be specified by the same method as described above to derive the wavelength variation from the initial central wavelength of the measurement light L, and the measurement value may be corrected based on the correlation between the wavelength variation and the variation of the measurement value, which are acquired in advance, to perform the quantification.

It should be noted that, in the color plate 75, it is preferable that the optical density changes by 0.2 or more in a region of ±40 nm with respect to the central wavelength of the light-emitting elements 73a and 73b, and it is more preferable that the optical density changes by 0.2 or more in a region of ±20 nm with respect to the central wavelength. This is because, in a case in which the optical density of the color plate 75 changes greatly before and after the central wavelength, the variation in the central wavelength can be detected with higher accuracy.

In a case in which the color plate of which the optical density changes by 0.2 or more in a wavelength range of 400 nm to 450 nm is provided as the color plate 75, the variation in the central wavelength can be accurately detected for the blue measurement light L having a wavelength of about 420 nm to 430 nm. In a case in which the color plate of which the optical density changes by 0.2 or more in a wavelength range of 500 nm to 580 nm is provided as the color plate 75, the variation in the central wavelength can be accurately detected for the green measurement light L having a wavelength of about 530 nm to 550 nm. Further, in a case in which the color plate of which the optical density changes by 0.2 or more in a wavelength range of 600 nm to 680 nm is provided as the color plate 75, the variation in the central wavelength can be accurately detected for the red measurement light L having a wavelength of about 630 nm to 650 nm.

In addition, in a case in which a plurality of light-emitting elements that emit the measurement light L in wavelength ranges different from each other are provided as the light-emitting elements 73a and 73b, and the color plate 75 includes a plurality of color plates of which the optical density changes in accordance with the wavelength range of each of the plurality of light-emitting elements, and the wavelength variation can be accurately detected for each measurement light of each wavelength.

Further, in the analysis apparatus 100, in a case in which the color plate 75 is defined as a first color plate (hereinafter, referred to as a first color plate 75), a second color plate 76 which is different from the first color plate 75 and used for correcting the illuminance variation, may be provided. The second color plate 76 is arranged within the imaging range and has a region irradiated with the measurement light L emitted from the light-emitting elements 73a and 73b. The second color plate 76 is a color plate of which the optical density changes by less than 0.2 in a wavelength range of 400 nm to 700 nm. The fact that, in the second color plate 76, the optical density changes by less than 0.2 in a wavelength range of 400 nm to 700 nm means that the difference ΔOD between the maximum value and the minimum value of the optical density is less than 0.2, and means that a change in optical density (that is, reflectivity) due to the wavelength variation is small. It should be noted that it is preferable that the second color plate 76 is a color plate of which the optical density changes by 0.1 or less in a wavelength range of 400 nm to 700 nm.

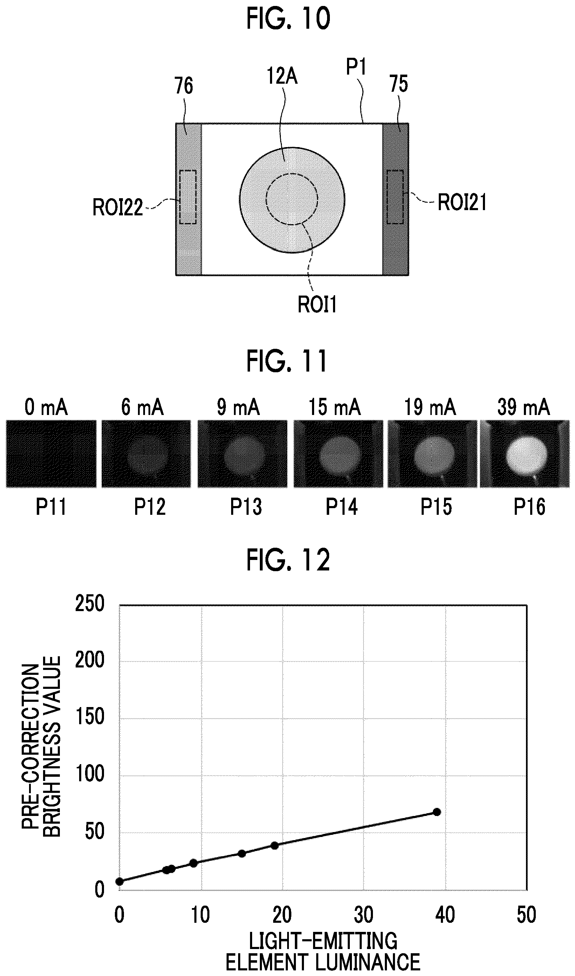

In a case in which the first color plate 75 and the second color plate 76 are provided, for example, as shown in Fig. 9, a U-shaped first color plate 75 and a U-shaped second color plate 76 are arranged between the photometry unit 70 and the analysis chip 12, instead of a rectangular color plate 75 having a rectangular opening in Fig. 5. Fig. 10 is a schematic diagram showing the image P1 acquired from the photometry unit 70 by the processor 90 in a form in which the second color plate 76 is provided. In the image P of Fig. 8, the color plates 75 are shown on both sides, but in the image P1 of Fig. 10, one (right side in Fig. 10) of the color plates 75 is the first color plate 75, and the other (left side in Fig. 10) thereof is the second color plate 76. It is preferable that the second color plate 76 has the optical density of 1.5 or less in a visible range (wavelength of 400 nm to 700 nm). It is preferable that the color plate 75 is a gray plate or a white plate.

As described above, in the configuration in which the second color plate 76 is provided in addition to the first color plate 75, the processor 90 specifies the wavelength of the measurement light L from the brightness value of the first color plate 75 in the image P1, extracts the brightness value of the second color plate 76 as the correction brightness value, corrects the measurement value based on the correction brightness value, and then derives the concentration of the detection target substance.

In a case in which the light-emitting elements 73a and 73b are, for example, light-emitting diodes (LEDs), the amount of light emitted may change due to a temperature change or the like. In a case in which the amount of light emitted by the light-emitting elements 73a and 73b changes, as a result, the illuminance of the reaction region 12A, the first color plate 75, and the second color plate 76 affected by the measurement light L changes. In a case in which the illuminance changes, the amount of reflection changes, and thus the brightness value (that is, the optical density) in the image P1 changes. In a case in which the optical density decreases due to the decrease in illuminance, an error occurs in the concentration of the detection substance specified from the optical density.

Therefore, in order to suppress the error caused by the change in illuminance, in the present configuration, a region of interest ROI22 of the second color plate 76 is set as the region for extracting the correction brightness data, and the processor 90 derives an average value B of the brightness data in this region as a correction brightness value (hereinafter, referred to as a correction brightness value B). Then, an average value A (hereinafter, a measurement value A) of the correction brightness data extracted from the region of interest ROI1 of the reaction region 12A, which is derived as the photometric region brightness value, is divided by the correction brightness value B to obtain A/B as a corrected measurement value. It should be noted that, here, the "brightness data" includes the brightness values of a plurality of pixels included in a certain region. Therefore, the average value of the brightness data is obtained by dividing the sum of the brightness values of the respective pixels included in the brightness data by the number of pixels. However, as the brightness value, instead of the average value of the brightness data, a median value of the brightness data may be used, or a most frequent value of the brightness data may be used.

Meanwhile, the processor 90 derives an average value C of the brightness data for detecting the wavelength variation extracted from a region of interest ROI21 of the first color plate 75 as the wavelength variation detection brightness value. Then, the processor 90 derives the reflectivity from the wavelength variation detection brightness value, and specifies the wavelength of the measurement light L from the relationship between the wavelength and the reflectivity of the incident light (see Fig. 6). As a result, the wavelength of the measurement light L during the analysis is specified with high accuracy. The processor 90 selects the calibration curve measured at the specified wavelength.

The processor 90 quantifies the concentration of the detection target substance from the selected calibration curve and the corrected measurement value A/B.

In this way, in the present embodiment, the image P1 including the reaction region 12A, the first color plate 75, and the second color plate 76 at the same time is acquired. From the image P1, the information on the measurement light L with which the reaction region 12A is irradiated can be acquired based on the first color plate 75 and the second color plate 76. By detecting the wavelength variation from the first color plate 75, selecting the calibration curve in accordance with the wavelength of the measurement light L, and quantifying the detection target substance from the calibration curve using the corrected measurement value A/B obtained by correcting the measurement value A with the correction brightness value B, it is possible to perform the quantitative analysis in which the error due to the wavelength variation and the change in illuminance is suppressed, and it is possible to obtain a more accurate analysis result.

Further, the processor 90 may correct the measurement value A based on a correlation between the correction brightness value B and, for example, the pre-photometric region brightness value and the pre-correction brightness value measured in advance using the reference plate. The pre-photometric region brightness value is a brightness value of a region corresponding to the reaction region 12A in the image captured by the photometry unit 70 in a state in which the reference plate is arranged at the measurement position instead of the analysis chip 12. The pre-correction brightness value is a brightness value of the second color plate 76 acquired from the image from which the pre-photometric region brightness value is acquired. The correlation between the pre-correction brightness value and the pre-photometric region brightness value is acquired by the adjustment operation of the processor 90, for example, during a period from the activation of the analysis apparatus 100 to the start of the light measurement by the photometry unit 70. As the reference plate, for example, a white plate W (see Fig. 2) for brightness calibration provided in the rotary substrate 62 need only be used.

The adjustment operation executed by the processor 90 is as described below.
First, the rotary substrate 62 is rotated to arrange the reference plate (for example, the white plate W) at the measurement position. The area sensor 74 acquires the image including at least a part of the reference plate and the second color plate 776 while changing the current value applied to the light-emitting elements 73a and 73b in a state in which the reference plate is arranged at the measurement position. In the image that is acquired here, the reference plate is observed from the opening window 62A of the rotary substrate 62. The light-emitting elements 73a and 73b are, as an example, LEDs. Fig. 11 is a diagram showing images P11 to P16 captured by applying the current values of 0 mA, 6 mA, 9 mA, 15 mA, 19 mA, and 39 mA to the light-emitting elements 73a and 73b. As shown in Fig. 11, the amount of light emission (irradiation amount) by the light-emitting elements 73a and 73b is larger as the current value is larger, and thus a bright (high-brightness) image is acquired.

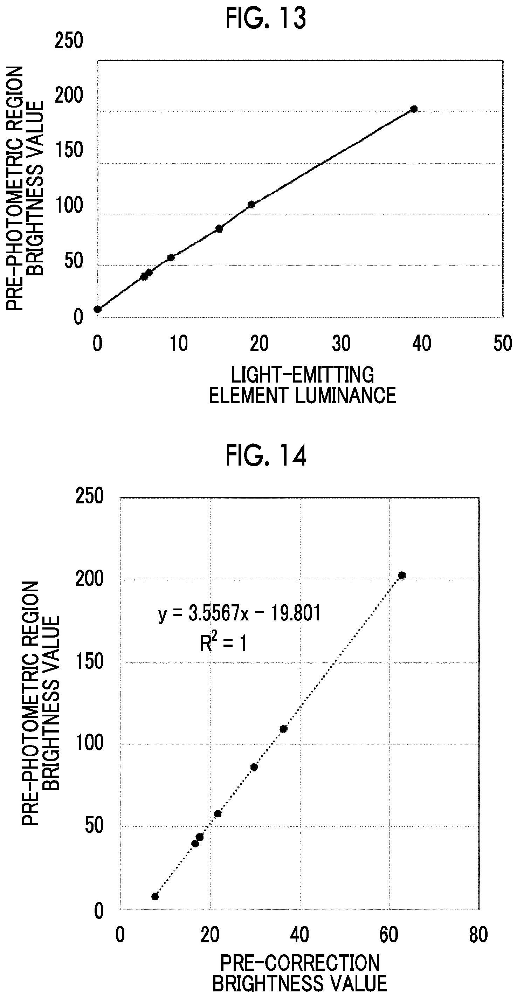

The correlation between the pre-photometric region brightness value and the pre-correction brightness value is obtained from the plurality of images P11 to P16 having different amounts of light emission of the light-emitting elements 73a and 73b shown in Fig. 11. The relationship between the light-emitting element illuminance and the pre-correction brightness value shown in Fig. 12 and the relationship between the light-emitting element illuminance and the pre-photometric region brightness value shown in Fig. 13 are derived. The pre-correction brightness value is derived from the brightness data of the region of interest ROI22 on the second color plate 76 from which the correction brightness data is extracted during the quantitative analysis in the image P1 schematically shown in Fig. 10. Similarly, the pre-photometric region brightness value is derived from the brightness data of the region (that is, the reference plate) corresponding to the region of interest ROI1 of the reaction region 12A from which the photometric region brightness data is extracted during the quantitative analysis in the image P1 schematically shown in Fig. 10.

Then, from the relationship shown in Figs. 12 and 13, the correlation between the pre-photometric region brightness value and the pre-correction brightness value shown in Fig. 14 is obtained. In the example shown in Fig. 14, the correlation between the pre-correction brightness value x and the pre-photometric region brightness value y is approximated by a linear function y = 3.5567x - 19.801 with a coefficient of determination R² = 1 (y = ax + b, where a = 3.5567 and b = -19.801). The processor 90 stores a relational expression between the pre-correction brightness value x and the pre-photometric region brightness value y in the memory. The processing of deriving the relational expression and storing the relational expression is processing executed in advance by the adjustment operation.

During the quantitative analysis, the processor 90 corrects the measurement value A, which is the photometric region brightness value, by using the above-described relational expression indicating the correlation between the pre-correction brightness value and the pre-photometric region brightness value acquired by the adjustment operation. Specifically, a value A/y obtained by dividing the measurement value A by the relational expression y is derived as the corrected measurement value. In a case in which the correlation between the pre-correction brightness value and the pre-photometric region brightness value is approximated by a linear function represented by a linear function y = ax + b as in Fig. 14, A/(ax + b) is derived as the corrected measurement value. In the example of Fig. 14, A/(3.5567 × B - 19.801) is derived. Here, B is the correction brightness value acquired from the color plate 75 in the image from which the measurement value A is acquired. Then, the processor 90 derives the optical density from the corrected measurement value, and derives the concentration of the detection target substance from the calibration curve based on the optical density obtained from the corrected measurement value.

Due to the deterioration of the light-emitting elements 73a and 73b, the illuminance distribution may change as the measurement is repeated, and the relationship between the illuminance in the reaction region and the illuminance in the second color plate 76 may change. However, as described above, in a case in which the processor 90 extracts the correction brightness value, which is the brightness value of the second color plate 76, from the image in addition to the photometric region brightness value and corrects the measurement value based on the correction brightness value and the correlation between the pre-photometric region brightness value and the pre-correction brightness value that are acquired in advance, the measurement value can be corrected with high accuracy even in a case in which a change in the illuminance distribution occurs due to the deterioration of the light-emitting elements 73a and 73b. As a result, the quantitative analysis with extremely high accuracy can be realized.

As described above, it is preferable that the optical density of the second color plate 76 is 1.5 or less. It is preferable that the second color plate 76 is gray or white. This is because the correction accuracy is improved in a case in which the amount of the reflected light is large and the brightness value of the second color plate 76 in the image P1 captured by the area sensor 74 is high.

The photometry unit 70 of the analysis apparatus 100 according to the above-described embodiment comprises the two light-emitting elements 73a and 73b that emit the light having the same central wavelength, but the number of light-emitting elements that emit the light having the same central wavelength may be three or more or may be one.

Further, in the above-described embodiment, various processors shown below can be used as the hardware structure of the processor 90. The various processors include, in addition to a CPU that is a general-purpose processor that executes software (program) to function as various processing units, a programmable logic device (PLD) of which a circuit configuration can be changed after manufacturing, such as a field-programmable gate array (FPGA), and a dedicated electric circuit that is a processor having a circuit configuration dedicatedly designed for executing specific processing, such as an application specific integrated circuit (ASIC).

The processing described above may be executed by one of the various processors or may be executed by a combination of two or more processors (for example, a combination of a plurality of FPGAs or a CPU and an FPGA) of the same type or different types. Moreover, a plurality of processing units may be configured by one processor. As an example in which the plurality of processing units are configured by one processor, there is a form in which a processor that realizes all functions of a system including the plurality of processing units by using one integrated circuit (IC) chip is used, such as a system on a chip (SOC).

Further, the hardware structure of these processors is, more specifically, an electric circuit (circuitry) in which the circuit elements, such as semiconductor elements, are combined.

In addition to the operation program of the analysis apparatus, the technology of the present disclosure extends to a computer readable storage medium (USB memory or digital versatile disc (DVD)-read only memory (ROM), or the like) that stores the operation program of the analysis apparatus in a non-transitory manner.

It should be noted that the above-described contents and the above-shown contents are for detailed description of the parts according to the technology of the present disclosure and are merely examples of the technology of the present disclosure. For example, the description of the configuration, the function, the operation, and the effect above are the description of examples of the configuration, the function, the operation, and the effect of the parts according to the technology of the present disclosure. As a result, it goes without saying that unnecessary parts may be deleted, new elements may be added, or replacements may be made with respect to the above-described contents and the above-shown contents within a range that does not deviate from the gist of the technology of the present disclosure. In addition, in order to avoid complication and facilitate understanding of the parts according to the technology of the present disclosure, the description related to common technical knowledge or the like that does not need to be particularly described for enabling implementation of the technology of the present disclosure is omitted in the above-described contents and the above-shown contents.

All of the documents, the patent applications, and the technical standards described in the present specification are incorporated into the present specification by reference to the same extent as in a case in which each of the documents, the patent applications, and the technical standards are specifically and individually stated to be described by reference.

In regard to the above-described embodiment, following supplementary notes are further disclosed.

### Supplementary Note 1

An analysis apparatus comprising: a support section that supports, at a measurement position, an analysis chip having a reaction region, which holds a reagent, and used for quantitative analysis of a detection target substance that reacts with the reagent; a photometry unit including a light-emitting element that irradiates the reaction region of the analysis chip with measurement light for measuring the reaction, and an area sensor that captures an image of a predetermined imaging range including the reaction region irradiated with the measurement light; and a color plate that is arranged within the imaging range, that has a region irradiated with the measurement light, that has characteristics in which a reflectivity changes in accordance with a wavelength of incident light, and that is used for detecting a wavelength variation of the measurement light.

### Supplementary Note 2

The analysis apparatus according to supplementary note 1, in which a central wavelength of the measurement light is in a wavelength range of 400 nm to 700 nm, and the color plate is used for detecting the wavelength variation of the measurement light emitted from the light-emitting element.

### Supplementary Note 3

The analysis apparatus according to supplementary note 2, in which the color plate has an optical density that changes by 0.6 or more in the wavelength range.

### Supplementary Note 4

The analysis apparatus according to supplementary note 2 or 3, in which the color plate has an optical density that changes by 0.2 or more in a region of ±40 nm with respect to a central wavelength of the light-emitting element.

### Supplementary Note 5

The analysis apparatus according to any one of supplementary notes 2 to 4, in which a plurality of light-emitting elements that emit the measurement light in wavelength ranges different from each other are provided as the light-emitting elements, the color plate includes a plurality of color plates of which an optical density changes in accordance with the wavelength range of each of the plurality of light-emitting elements.

### Supplementary Note 6

The analysis apparatus according to any one of supplementary notes 2 to 5, in which the color plate includes a color plate of which an optical density changes by 0.2 or more in a wavelength range of 400 nm to 450 nm.

### Supplementary Note 7

The analysis apparatus according to any one of supplementary notes 2 to 6, in which the color plate includes a color plate of which an optical density changes by 0.2 or more in a wavelength range of 500 nm to 580 nm.

### Supplementary Note 8

The analysis apparatus according to any one of supplementary notes 2 to 7, in which the color plate includes a color plate of which an optical density changes by 0.2 or more in a wavelength range of 600 nm to 680 nm.

### Supplementary Note 9

The analysis apparatus according to any one of supplementary notes 2 to 8, in which, in a case in which the color plate is defined as a first color plate, a second color plate different from the first color plate is provided, and the second color plate is arranged within the imaging range, has a region irradiated with the measurement light emitted from the light-emitting element, and has an optical density that changes by less than 0.2 in a wavelength range of 400 nm to 700 nm.

### Supplementary Note 10

The analysis apparatus according to supplementary note 9, in which the optical density of the second color plate is 1.5 or less.

### Supplementary Note 11

The analysis apparatus according to any one of supplementary notes 1 to 10, in which the analysis chip contains a dry reagent as the reagent.

### Supplementary Note 12

The analysis apparatus according to any one of supplementary notes 1 to 11, further comprising: a processor that acquires the image from the photometry unit to perform the quantitative analysis of the detection target substance based on a measurement value corresponding to a photometric region brightness value that is a brightness value of the reaction region extracted from the acquired image, in which the processor detects the wavelength variation of the measurement light based on the image to perform the quantitative analysis in accordance with the detected wavelength variation.

### Explanation of References

10: chip set section
11: holding table
12: analysis chip
12A: reaction region
14: stocker
16: carrier
17: case
17A: first case
17B: second case
17C: opening
17D: opening
17E: information code
20: reader
30: specimen spotting section
31: chip support table
40: chip transport mechanism
42: chip transport member
44: drive mechanism
50: specimen spotting mechanism
52: nozzle
60: incubator
62: rotary substrate
62A: opening window
64: pressing member
64A: pressing surface
65: holding member
66: rotary cylinder
67: bearing
68: disposal hole
69: thermal insulation cover
70: photometry unit
71: housing
73: irradiation device
73a, 73b: light-emitting element
74: area sensor
75: color plate (first color plate)
76: second color plate
80: chip disposal mechanism
82: chip transport member
84: drive mechanism
90: processor
100: analysis apparatus
L: measurement light
L1: reflected light
P, P1: image
P11 to P16: image
ROI1: region of interest of reaction region
ROI2: region of interest of color plate 75
ROI3: region of interest of color plate 75
ROI21: region of interest of first color plate 75
ROI22: region of interest of second color plate 76
S: cell

## Claims

1. An analysis apparatus (100) comprising:
a support section (62) that supports, at a measurement position, an analysis chip (12) having a reaction region (12A), which holds a reagent, and used for quantitative analysis of a detection target substance that reacts with the reagent;
a photometry unit (70) including a light-emitting element (73a, 73b) that irradiates the reaction region of the analysis chip with measurement light for measuring the reaction, and an area sensor (74) that captures an image of a predetermined imaging range including the reaction region irradiated with the measurement light; and
a color plate (75) that is arranged within the imaging range, that has a region irradiated with the measurement light, that has characteristics in which a reflectivity changes in accordance with a wavelength of incident light, and that is used for detecting a wavelength variation of the measurement light.

2. The analysis apparatus (100) according to claim 1,
wherein a central wavelength of the measurement light is in a wavelength range of 400 nm to 700 nm, and
the color plate (75) is used for detecting the wavelength variation of the measurement light emitted from the light-emitting element.

3. The analysis apparatus (100) according to claim 2,
wherein the color plate (75) has an optical density that changes by 0.6 or more in the wavelength range.

4. The analysis apparatus (100) according to claim 2 or 3,
wherein the color plate (75) has the optical density that changes by 0.2 or more in a region of ±40 nm with respect to a central wavelength of the light-emitting element.

5. The analysis apparatus (100) according to any one of claims 2 to 4,
wherein a plurality of light-emitting elements that emit the measurement light in wavelength ranges different from each other are provided as the light-emitting elements (73a, 73b), and
the color plate (75) includes a plurality of color plates of which the optical density changes in accordance with the wavelength range of each of the plurality of light-emitting elements.

6. The analysis apparatus (100) according to any one of claims 2 to 5,
wherein the color plate (75) includes a color plate of which the optical density changes by 0.2 or more in a wavelength range of 400 nm to 450 nm.

7. The analysis apparatus (100) according to any one of claims 2 to 6,
wherein the color plate (75) includes a color plate of which the optical density changes by 0.2 or more in a wavelength range of 500 nm to 580 nm.

8. The analysis apparatus (100) according to any one of claims 2 to 7,
wherein the color plate (75) includes a color plate of which the optical density changes by 0.2 or more in a wavelength range of 600 nm to 680 nm.

9. The analysis apparatus (100) according to any one of claims 2 to 8,
wherein, in a case in which the color plate (75) is defined as a first color plate, a second color plate (76) different from the first color plate is provided, and
the second color plate (76) is arranged within the imaging range, has a region irradiated with the measurement light emitted from the light-emitting element, and has an optical density that changes by less than 0.2 in a wavelength range of 400 nm to 700 nm.

10. The analysis apparatus (100) according to claim 9,
wherein the optical density of the second color plate (76) is 1.5 or less.

11. The analysis apparatus (100) according to any one of claims 1 to 10,
wherein the analysis chip contains a dry reagent as the reagent.

12. The analysis apparatus (100) according to any one of claims 1 to 11, further comprising:
a processor (90) that acquires the image from the photometry unit (70) to perform the quantitative analysis of the detection target substance based on a measurement value corresponding to a photometric region brightness value that is a brightness value of the reaction region extracted from the acquired image,
wherein the processor (90) detects the wavelength variation of the measurement light based on the image to perform the quantitative analysis in accordance with the detected wavelength variation.
